# EUROPEAN PATENT SPECIFICATION

(11) **EP 1 267 869 B1**
(45) Date of publication and mention of the grant of the patent: **19.05.2004**
(21) Application number: 01910051.0
(22) Date of filing: 09.03.2001
(51) Int. Cl.: A61K 31/44, A61K 31/197, A61K 45/06, A61P 25/32, A61P 25/34, A61P 25/36, A61P 25/30, A61K 45/00

(54) **METABOTROPIC GLUTAMATE RECEPTOR ANTAGONISTS FOR THE TREATMENT OF TOLERANCE AND DEPENDANCE**
METABOTROPE GLUTAMATREZEPTORANTAGONISTEN ZUR BEHANDLUNG VON TOLERANZ UND ABHÄNGIGKEIT
ANTAGONISTE DU RECEPTEUR DE GLUTAMATE POUR TRAITEMENT D'ACCOUTUMANCE OU DE DEPENDANCE

(30) Priority: 09.03.2000 GB 0005700
(43) Date of publication of application: 02.01.2003
(73) Proprietor: GLAXO GROUP LIMITED, Greenford, Middlesex UB6 ONN (GB)
(72) Inventor: CORSI, Mauro, I-37100 Verona (IT); CONQUET, François Inst. Bio. Cel. Morphologie, 1005 Lausanne (CH)
(74) Representative: Thornley, Rachel Mary
(86) International application number: PCT/GB2001/001058
(87) International publication number: WO 2001/066113

(56) References cited:
- EP-A- 0 807 621
- WO-A-00/20001
- WO-A-00/63166
- WO-A-00/69816
- WO-A-98/40407
- WO-A-99/02497
- GHASEMZADEH M BEHNAM ET AL: "Neuroadaptations in ionotropic and metabotropic glutamate receptor mRNA produced by cocaine treatment." JOURNAL OF NEUROCHEMISTRY, vol. 72, no. 1, January 1999 (1999-01), pages 157-165, XP001000639 ISSN: 0022-3042

## Description

### Field of the invention

The invention relates to tolerance and dependence therapy. It also relates to screening methods for identifying new products which can be used in tolerance and dependence therapy.

### Background to the invention

Addiction is a chronic brain disease manifested by humans in a variety of behaviours and in a range of social circumstances. Although it is a complex phenomenon, its medical definition is a central nervous system (CNS) disorder manifested as a behavioural disturbance due to a neurobiological imbalance in the brain (Leshner, 1997, Science **278**, 45). Individuals may become addicted to a wide variety of factors, including substances like drugs. The obsessive and compulsive aspect of drug dependence may overlap with other obsessive compulsive behaviours such as gambling or compulsive sexual activity.

In respect of substance abuse, addict behaviour is induced and maintained in a multifactorial fashion with a central role played by the unconditioned reinforcing properties of the abused drug. There are many different substances on which individuals may become dependent, including opiates, benzodiazepines, amphetamine, nicotine, cocaine and ethanol.

The impact of substance dependence is huge. For example, nicotine dependence is the most widely diffused type of drug addiction. One third of the worldwide population over 15 years of age are smokers. Smoking continues to increase among adolescents and by the year 2025 the WHO estimates that there will be 10 million tobacco related deaths per year. Stopping smoking may evoke a range of symptoms in dependent individuals, including craving, depression, anxiety, difficulty in concentrating and weight gain. Despite a variety of available treatments many smokers fail to give up smoking.

There is therefore a major unmet need in the area of substance abuse for pharmacological agents that are more effective that those currently available at reducing withdrawal symptoms and more importantly reducing relapse rates. Indeed smoking cessation is a therapeutic area with generally poor results: an avearage 30% success rate compared with 50 to 80% for alcoholism, opioid and cocaine dependence (at 6 months).

Nevertheless the rationale for pharmacological intervention is, however, still strong because only pharmacotherapy potentially acts on a population larger than that treated with psychosocial interventions and therefore may enhance these traditional methods by improving compliance and quality of the treatment.

Glutamate is the transmitter of the vast majority of excitatory synapses in the mammalian CNS and plays an important role in a wide variety of CNS functions. In the past, the actions of glutamate in the mammalian brain were thought to be mediated exclusively by activation of glutamate-gated cation channels termed ionotropic glutamate receptors: see Watkins & Evans, Ann. Rev. Pharmacol. Toxicol., **21**, 165 (1985). In the mid-1980s, however, evidence for the existence of glutamate receptors directly coupled to a second messenger via G-proteins began to appear with the discovery of a glutamate receptor coupled to activation of phosphonoinositide hydrolysis. That led to the discovery of a new family of glutamate receptors named metabotropic glutamate receptors (mGluRs): see for example, Sladeczek *et al*., Nature, **317**, 717 (1985) and Sugiyama *et al*., Nature, **325**, 531 (1987).

The search for mGluR-related cDNAs has resulted in the isolation of eight genes that encode distinct mGluRs. These receptors are named mGluR1 through mGluR8. Based on their amino acid sequence identity, the eight mGluRs can be classified into three groups. Group I includes mGluR1 and mGluR5, group II mGluR2 and mGluR3 and group III mGluR4, mGluR6, mGluR7 and mGluR8. Whereas group I mGluRs stimulate inositol phosphate metabolism and mobilization of intracellular Ca²⁻, both group II and group III mGluRs are negatively coupled to adenylyl cyclase (Schoepp & Conn, Trend Pharmacol. Sci., **14**, 13, 1993 and Pin & Duvoisin, Neuropharmacology, 34, 1 (1995)).

WO 98140407 discloses a new family of Synaptic activation proteins (imparticular Homer) that were found to bind mGluR5 or mGluR1x. It is suggested that these proteins could be used in screening assays to identity compounds that interfere with or modulate, this binding, which compounds could perhaps be used as drugs in treating epilepsy, abnormal brain development, neural injury trauma or certain chemical additions. There is no indication as to whether such compounds would themselves be agonists or antagonists of the receptor activity, or whether they would have any effect at all other than to interfere with Homer's binding to the receptor.

### Summary of the invention

This invention is based on our findings that:
(i) a compound which can act as a selective antagonist of the metabotropic glutamate receptor, mGluR5, can reduce the reinstatement of nicotine-seeking behaviour in rats following exposure to experimental determinants of smoking relapse; and
(ii) mGluR5 knockout mice do not display cocaine-induced hyperactivity and show no response to the reinforcing properties of cocaine and do not self-administer amphetamine at any tested dose.

We propose that pharmacological block of mGluR5 leads to negative regulation of dopamine-2 (D2) receptors, which in turn reduces dopaminergic activity. The reduction in dopaminergic activity leads to a reduction in smoking relapse. We also propose that mGluR5 is responsible for the hyperactive response to cocaine administration and is also an essential component of the reward process induced by cocaine and amphetamine.

Furthermore, we propose that mGluR5 is involved in "emotional learning". Addiction implies that an individual has first "learned" how to be dependent before being addicted. Every memorisation process is preceded and we suggest that mGluR5 is responsible for the "learning" process of dependence, regardless of the type of dependence in question. The mGluR5 receptor is thus required for the onset of the dependence process, before the final settlement of physiological addiction.

According to the present invention there is thus provided use of an antagonist of mGluR5, typically human mGluR5, in the manufacture of a medicament for use in a method of tolerance or dependence therapy.

The invention also provides:
- an antagonist of mGluR5 for use in a method of treatment of the human or animal body by therapy;
- a method of treating a host suffering from tolerance or dependence, which method comprises administering to the host a therapeutically effective amount of an antagonist of mGluR5;
- a pharmaceutical composition comprising an antagonist of mGluR5 and a pharmaceutically acceptable carrier or diluent;
- products containing an antagonist of mGluR5 and a therapeutic substance as a combined preparation for simultaneous, separate or sequential use in the treatment of a condition for which the said therapeutic substance is used, wherein the use of the therapeutic substance in the absence of said antagonist could lead to tolerance of or dependence on the therapeutic substance;
- use of mGluR5 for identifying a product for use in the treatment tolerance or dependence;
- a method for identifying a product for use in the treatment of tolerance or dependence, comprising:
   (a) contacting a test product with mGluR5 under conditions that in the absence of the test substance would lead to activity of the said mGluR5, and
   (b) determining whether the test product antagonises mGluR5 activity, thereby to determine whether the test product may be used in the treatment of substance tolerance or dependence;
- a product identified by a method of the invention;
- a product of the invention for use in a method of treatment of the human or animal body by therapy;
- use of a product of the invention for the manufacture of a medicament for use in tolerance or dependence therapy;
- a method of treating a host suffering from tolerance or dependence, which method comprises administering to the host a therapeutically effective amount of a product of the invention;
- a pharmaceutical composition comprising a product of the invention and a pharmaceutically acceptable carrier or diluent; and
- products containing a product of the invention and a therapeutic substance as a combined preparation for simultaneous, separate or sequential use in the treatment of a condition for which the said therapeutic substance is used, wherein the use of the therapeutic substance in the absence of said product could lead to tolerance of or dependence on the therapeutic substance.

### Brief description of the figures

Figure 1
   (a) shows the effects of cocaine on mutant (●) and wild-type mice (■) mice motor activity. Horizontal activity was measured every 5 minutes during a 60-min session. Mice were injected with saline i.p. and placed in the apparatus at time 0. At time 15 min, mice were injected with cocaine 10 mg/kg i.p. and placed again in the apparatus. Statistics (oneway ANOVA; n = 5-12) was performed by comparison of values from time bin 20 to 60 min. SEM are omitted.
   (b) shows the effects of cocaine on mutant (filled bars) and wild-type mice (open bars) motor activity. The total amount of horizontal activity measure during the 60-min session was calculated as percentage of vehicle treatment effect (% counts vs vehicle). Mice were injected with saline i.p. and placed in the apparatus at time 0. At time 15 min, mice were injected with cocaine 10, 20 or 40 mg/kg i.p. and placed again in the apparatus. Statistics (oneway ANOVA followed by Dunnett's;
   * = P<0.05; n = 14-16) were performed by comparison of values from time bin 20 to 60 min. SEM are omitted.
Figure 2 shows the results of cocaine self-administration experiments;
   (a) Learing task experiment with food reinforcer (sugar milk) shows that both mGlu(+/+) (filled bar) and (-/-) (open bar) performed an equal operant behaviour; and
   (b) Dose response curve at different doses of cocaine in self-administration paradigm for wild type (■) and knockout (○)mice.
Figure 3 shows the results of dopamine microdialysis in the nucleus accumbens of (a) wild type and (b) knockout mice after injection of 10mg/kg of cocaine or saline buffer intraperiteonally.
Figure 4 shows d-amphetamine self-administration in mGluR5 knock-out (-/-) mice.

### Detailed description of the invention

The present invention is concerned with antagonists of mGluR5 for treating tolerance or dependence. The mGluR5 is preferably human mGluR5.

An antagonist of mGluR5 is a substance which diminishes or abolishes the effect of a ligand (agonist) which typically activates mGluR5. Thus, the antagonist may be, for example, a chemical antagonist, a pharmacokinetic antagonist, an antagonist by receptor block, a non-competitive antagonist or a physiological antagonist.

A chemical antagonist is wherein the antagonist binds the ligand in solution so the effect of the ligand is lost.

A pharmacokinetic antagonist is one which effectively reduces the concentration of the active drug at its site of action, for example by increasing the rate of metabolic degradation of the active ligand.

Antagonism by receptor-block involves two important mechanisms:
reversible competitive antagonism and irreversible, or non-equilibrium, competitive antagonism. Reversible competitive antagonism occurs when the rate of dissociation of the antagonist molecules is sufficiently high that, on addition of the ligand, displacement of the antagonist molecules from the receptors effectively occurs. Of course the ligand cannot evict a bound antagonist molecule, or *vice versa*. Irreversible or non-equilibrium, competitive antagonism occurs when the antagonist dissociates very slowly, or not at all, from the receptor with the result that no change in the antagonist occupancy takes place when the ligand is applied. Thus the antagonism is insurmountable.

Non-competitive antagonism describes the situation where the antagonist blocks at some point in the signal transduction pathway leading to the production of a response by the ligand.

Physiological antagonism is a term used loosely to describe the interaction of two substances whose opposing actions in the body tend to cancel each other out.

An antagonist can also be a substance which diminishes or abolishes expression of functional mGluR5. Thus an antagonist can be, for example, a substance which diminishes or abolishes expression of the gene encoding mGluR5, diminishes or abolishes translation of mGluR5 RNA, diminishes or abolishes post-translational modification of mGluR5 protein or diminishes or abolishes the insertion of mGluR5 into the cell membrane.

Preferred antagonists are those which lead to a reduction of activation by the ligand of at least 10%, at least 20%, at least 30%, at least 40%, at least 50%, at least 60%, at least 70%, at least 80%, at least 90%, at least 95% or at least 99% at a concentration of the antagonist of 1 µgml⁻¹, 10 µgml⁻¹, 100 µgml⁻¹, 500 µgml⁻¹, 1 mgml⁻¹, 10 mgml⁻¹, 100mg ml⁻¹. The percentage antagonism represents the percentage decrease in activity of mGluR5 in a comparison of assays in the presence and absence of the antagonist. Any combination of the above mentioned degrees of percentage antagonism and concentration of antagonist may be used to define an antagonist of the invention, with greater antagonism at lower concentrations being preferred.

An antagonist for use in the invention may be a relatively non-specific antagonist which is an antagonist of mGluRs in general. Preferably, however, an antagonist antagonises only group I mGluRs. More preferably, an antagonist used in the invention is a selective antagonist of mGluR5. A selective antagonist of mGluR5 is one which antagonises mGluR5, but antagonises other mGluRs only weakly or substantially not at all. Most preferred antagonists are those which can selectively antagonise mGluR5 at low concentrations, for example those that cause a level of antagonism of 50% or greater at a concentration of 100 µgml⁻¹ or less.

Selective mGluR5 antagonists can thus typically exhibit at least 100 fold greater activity at an mGluR5 receptor than at an mGluR1 receptor, preferably at least 200 fold greater activity and most preferably at least 400 fold greater activity. They can display a high degree of selectivity and affinity as antagonists of the human and/or rat mGluR5.

Suitable antagonists for use in the invention are disclosed in EP-A-0807621, WO 99/02497, WO 00/20001 and WO 00/63166. As disclosed in WO 99/02497, therefore, suitable antagonists may thus have the formula (I): 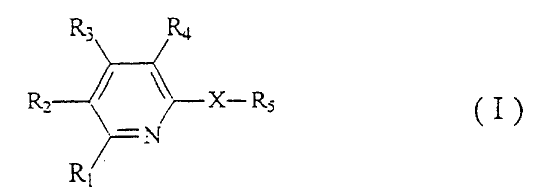 wherein
R₁ denotes hydrogen, lower alkyl, hydroxy-lower alkyl, lower alhyl-amino, piperidino, carboxy, esterified carboxy, amidated carboxy, unsubstituted or lower alkyl-, lower alkoxy-, halo- and/or trifluoromethyl-substituted N-lower-alkyl-N-phenylcarbamoyl, lower alkoxy, halo-lower alkyl or halo-lower alkoxy;
R₂ denotes hydrogen, lower alkyl, carboxy, esterified carboxy, amidated carboxy, hydroxy-lower alkyl, hydroxy, lower alkoxy or lower alkanoyloxy, 4-(4-fluoro-benzoyl)-piperidin-1-ylcarboxy, 4-t.butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy;
R₃ represents hydrogen, lower alkyl, carboxy, lower alkoxy-carbonyl, lower alkyl-carbamoyl, hydroxy-lower alkyl, di-lower alkyl-aminomethyl, morpholinocarbonyl or 4-(4-fluoro-benzoyl)-piperadin-1-yl-carboxy;
R₄ represents hydrogen, lower alkyl, hydroxy, hydroxy-lower alkyl, amino-lower alkyl, lower alkylamino-lower alkyl, di-lower alkylamino-lower alkyl, unsubstituted or hydroxy-substituted lower alkyleneamino-lower alkyl, lower alkoxy, lower alkanoyloxy, amino-lower alkoxy, lower alkylamino-lower alkoxy, di-lower alkylaino-lower alkoxy, phthalimido-lower alkoxy, unsubstituted or hydroxy- or 2-oxo-imidazolidin-1-yl-substituted lower alkyleneamino-lower alkoxy, carboxy, esterified or amidated carboxy, carboxy-lower alkoxy or esterified carboxy-lower alkoxy;
X represents an optionally halo-substituted lower alkenylene or alkynylene group bonded via vicinal saturated carbon atoms or an azo (-N=N-) group, and R₅ denotes an aromatic or heteroaromatic group which is unsubstituted or substituted by one or more substituents selected from lower alkyl, halo, halo-lower alkyl, halo-lower alkoxy, lower alkenyl, lower alkynyl, unsubstituted or lower alkyl-, lower alkoxy-, halo- and/or trifluoromethyl-substituted phenyl, unsubstituted or lower alkyl-, lower alkoxy-, halo and/or trifluoromethyl-substituted phenyl-lower alkynyl, hydroxy, hydroxy-lower alkyl, lower alkanoyloxy-lower alkyl, lower alkoxy, lower alkenyloxy, lower alkylenedioxy, lower alkanoyloxy, amino-, lower alkylamino-, lower alkanoylamino- or N-lower alkyl-N-lower alkanoylamino-lower alkoxy, unsubstiruted or lower alkyl-, lower alkoxy-, halo- and/or trifluoromethyl-substituted phenoxy, unsubstituted or lower alkyl-, lower alkoxy-, halo and/or trifluoromethyl-substituted phenyl-lower alkoxy, acyl, carboxy, esterified carboxy, amidated carboxy, cyano, carboxy-lower alkylamino, esterified carboxy-lower alkylamino, amidated carboxy-lower alkylamino, phosphono-lower alkylamino, esterified phosphono-lower alkylamino, nitro, amino, lower alkylamino, di-lower alkylamino-acylamino, N-acyl-N-lower alkylamino, phenylamino, phenyl-lower alkylamino cycloalkyl-lower alkylamino or heteroaryl-lower alkylamino each of which may be unsubstituted or lower alkyl-, lower alkoxy-, halo- and/or trifluoromethyl-substituted; their N-oxides and their pharmaceutically acceptable salts.

Compounds of formula (I) which have basic groups may form acid addition salts, and compounds of the formula (I) having acidic groups may form salts with bases. Compounds of formula (I) having basic groups and in addition having at least one acidic group, may also form internal salts. Also included are both total and partial salts, that is to say salts with 1, 2 or 3, preferably 2, equivalents of base per mole of acid of formula (I), or salts with 1, 2 or 3 equivalents, preferably 1 equivalent, of acid per mole of base of formula (I). Only the pharmaceutically acceptable, non-toxic salts are used therapeutically and they are therefore preferred.

Halo in the present description denotes fluorine, chlorine, bromine or iodine. Lower alkyl is typically C₁₋₆, for example C₁₋₄, alkyl. Lower alkoxy is typically C₁₋₆, for example C₁₋₄, alkoxy. Lower alkynyl is typically C₂₋₅ alkynyl. Lower alkanoyl is typically C₂₋₅ alkanoyl. Lower alkylene is typically C₂₋₅ alkylene. Lower alkenylene is typically C₂₋₅ alkenylene. Lower alkynylene is typically C₂₋₄ alkynylene.

When X represents an alkenylene group, configuration trans is preferred.

Preferred compounds of formula (I) are those wherein:
X represents an optionally halo-substituted (C₂₋₄)alkenylene or alkynylene group bonded via vicinal unsaturated carbon atoms.
R₁ is hydrogen, (C₁₋₄) alkyl, (C₁₋₄)alkoxy, hydroxy(C₁₋₄)alkyl, cyano, ethynyl, carboxy, (C₁₋₄)alkoxycarbonyl, di(C₁₋₄)alkylamino, (C₁₋₆)alkylaminocarbonyl, trifluoromethylphenylaminocarbonyl,
R₂ is hydrogen, hydroxy (C₁₋₄)alkyl, hydroxy (C₁₋₄)alkyl, (C₁₋₄)alkoxy, carboxy, (C₂₋₅)alkanoyloxy, (C₁₋₄)alkoxycarbonyl, di(C₁₋₄)alkylamino(C₁₋₄)alkanoyl, di(C₁₋₄)alkylaminomethyl, 4-(4-fluoro-benzoyl)-piperidin-1-yl-carboxy, 4-t.-butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy,
R₃ is hydrogen, (C₁₋₄) alkyl, carboxy, (C₁₋₄)alkoxycarbonyl, (C₁₋₄)alkylcarbamoyl, hydroxy(C₁₋₄)alkyl, di(C₁₋₄)alkylaminomethyl, morpholinocarbonyl or 4-(4-fluoro-benzoyl)-piperidin-1-yl-carboxy,
R₄ is hydrogen, hydroxy, (C₁₋₄)alkoxy, carboxy, (C₂₋₅)alkanoyloxy, (C₁₋₄)alkoxy-carbonyl, amino(C₁₋₄)alkoxy, di(C₁₋₄)alkylamino(C₁₋₄)alkoxy, di(C₁₋₄)alkylamino(C₁₋₄)alkyl, carboxy (C₁₋₄)alkylcarbonyl, (C₁₋₄)alkoxycarbonyl(C₁₋₄)alkoxy, hydroxy(C₁₋₄)alkyl, di(C₁₋₄)alkylamino(C₁₋₄)alkoxy, m-hydroxy-p-azidophenylcarbonylamino(C₁₋₄)alkoxy, and
R₅ is a group of formula 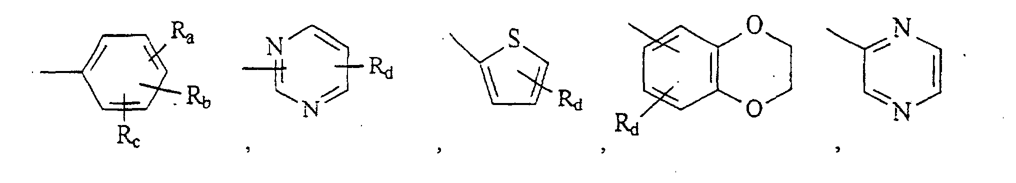 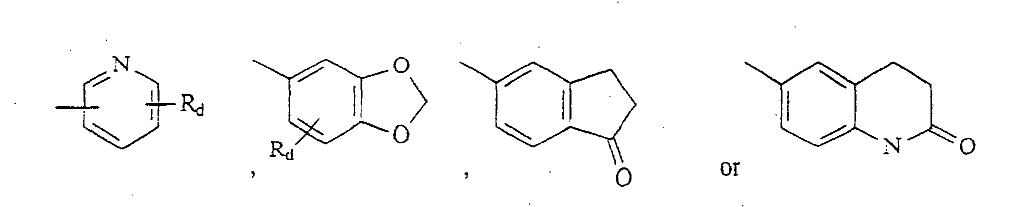 wherein:
Rₐ and R_{b} independently are hydrogen, hydroxy, halogen, nitro, cyano, carboxy, (C₁₋₄)alkyl, (C₁₋₄)alkoxy, hydroxy(C₁₋₄)alkyl, (C₁₋₄)alkoxycarbonyl, (C₂₋₇)alkanoyl, (C₂₋₅)alkanoyloxy, (C₂₋₅)alkanoyloxy(C₁₋₄)alkyl, trifluoromethyl, trifluoromethoxy, trimethylsilylethynyl, (C₂₋₅)alkynyl, amino, azido, amino (C₁₋₄)alkoxy, (C₂₋₅)alkanoylamino(C₁₋₄)alkoxy, (C₁₋₄)alkylamino(C₁₋₄)alkoxy, di(C₁₋₄)alkylamino(C₁₋₄)alkoxy, (C₁₋₄)alkylamino, di(C₁₋₄)alkylamino, monohalobenzylamino, thienylmethylamino, thienylcarbonylamino, trifluoromethylphenylaminocarbonyl, tetrazolyl, (C₂₋₅)alkanoylamino, benzylcarbonylamino, (C₁₋₄)alkylaminocarbonylamino, (C₁₋₄)alkoxycarbonylaminocarbonylamino or (C₁₋₄)alkylsulfonyl,
R_{c} is hydrogen, fluorine, chlorine, bromine, hydroxy, (C₁₋₄)alkyl, (C₂₋₅)alkanoyloxy, (C₁₋₄)alkoxy or cyano, and
R_{d} is hydrogen, halogen or (C₁₋₄)alkyl.

More preferred compounds of fomula (I) are those wherein X is as defined above and
R₁ is hydrogen, (C₁₋₄)alkyl, (C₁₋₄)alkoxy, cyano, ethynyl or di(C₁₋₄)alkylamino,
R₂ is hydrogen, hydroxy, carboxy, (C₁₋₄) alkoxycarbonyl, di(C₁₋₄)alkylaminomethyl, 4-(4-fluoro-benzoyl)-piperdin-1-yl-carboxy, 4-t.-butyloxycarbonyl-piperizin-1-yl carboxy, 4-(4-azido-2-hydroxybenzoyl)-pipeazin-1-yl-carboxy or 4-(4-azido-2-hydroxy-3-iodo-benzoyl)-piperazin-1-yl-carboxy,
R₃ is as defined above,
R₄ is hydrogen, hydroxy, carboxy, (C₂₋₅)alkanoyloxy, (C₁₋₄)alkoxycarbonyl, amino(C₁₋₄)alkoxy, di(C₁₋₄)alkylamino(C₁₋₄)alkoxy, di(C₁₋₄)alkylamino(C₁₋₄)alkyl or hydroxy(C₁₋₄)alkyl, and
R₅ is a group of formula 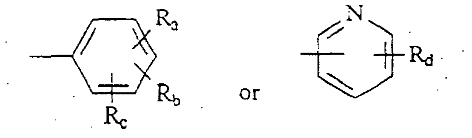 wherein:
Rₐ and R_{b} independently are hydrogen, halogen, nitro, cyano, (C₁₋₄)alkyl, C(₁₋₄)alkoxy, trifluoromethyl, trifluoromethoxy or (C₂₋₅)alkynyl, and R_{c} and R_{d} are as defined above.

Further selective mGluR antagonists are 2-arylalkenyl-, 2-heteroarylalkenyl-, 2-arylalkynyl-, 2-heteroaryl-alkynyl, 2-arylazo- and 2-heteroarylazo- pyridines, more particularly 6-methyl-2-(phenylazo)-3-pyridinol, (E)-2-methyl-6-styryl-pyridine and (phenylazo)-3-pyridinol, (E)-2-methyl-6-styryl-pyridine and compounds of formula (II): 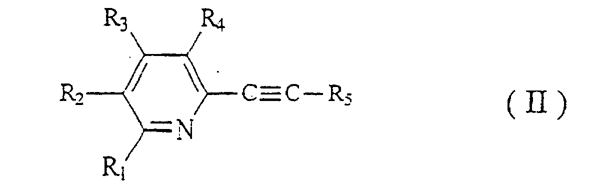 wherein
R₁ is hydrogen, (C₁₋₄)alkyl, (C₁₋₄)alkoxy, cyano, ethynyl or di(C₁₋₄)alkylamino,
R₂ is hydrogen, hydroxy, carboxy, (C₁₋₄) alkoxycarbonyl, di(C₁₋₄)alkylaminomethyl, 4-(4-fluoro-benzoyl)-piperidin-1-yl-carboxy, 4-t.butyloxycarbonyl-piperazin-1-yl-carboxy, 4-(4-azido-2-hydroxybenzoyl)-piperazin-1-yl-carboxy or 4-(4-azido-2-hydroxy-3-iodo-benzoyl)-piperazin-1-yl-carboxy.
R₃ is hydrogen, (C₁₋₄)alkyl, carboxy, (C₁₋₄)alkoxycarbonyl, (C₁₋₄)alkylcarbamoyl, hydroxy(C₁₋₄)alkyk, di(C₁₋₄)alkylaminomethyl, morpholinocarbonyl or 4-(4-fluoro-benzoyl)-piperazin-1-yl-carboxy,
R₄ is hydrogen, hydroxy, carboxy, C(₂₋₅)alkanoyloxy, (C₁₋₄)alkoxycarbonyl, amino (C₁₋₄)alkoxy, di(C₁₋₄)alkylamino(C₁₋₄)alkoxy, di(C₁₋₄)alkylamino(C₁₋₄)alkyl or hydroxy(C₁₋₄)alkyl, and
R₅ is a group of formula 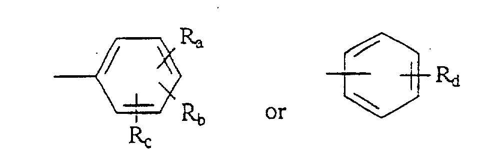 wherein
Rₐ and R_{b} independently are hydrogen, halogen, nitro, cyano, (C₁₋₄)alkyl, (C₁₋₄)alkoxy, trifluoromethyl, trifluoromethoxy or (C₂₋₅)alkynyl, and
R_{c} is hydrogen, fluorine, chlorine bromine, hydroxy (C₁₋₄)alkyl, C(₂₋₅)alkanoyloxy, (C₁₋₄)alkoxy or cyano; and
R_{d} is hydrogen, halogen or (C₁₋₄)alkyl;
in free form or in form of pharmaceutically acceptable salts.

Suitable phenyl glycine compounds are disclosed in EP-A-0807621. Phenyl glycine compounds useful in the invention can thus have the formula (III): 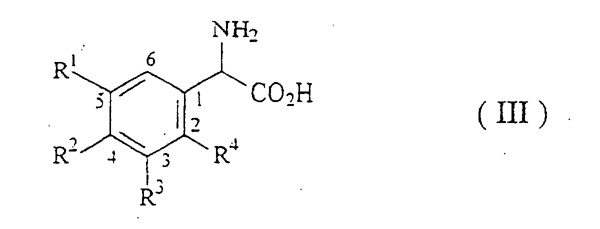 in which R¹ is hydrogen, hydroxy or C₁₋₆ alkoxy;
R₂ is hydrogen, carboxy, tetrazolyl, -SO₂H, -SO₃H, -OSO₃H, -CONHOH, or -P(OH)OR', -PO(OH)OR', -OP(OH)OR' or -OPO(OH)OR' where R' is hydrogen, C₁₋₆ alkyl, C₂₋₆ alkenyl or aryl C₁₋₆ aryl;
R₃ is hydrogen, hydroxy or C₁₋₄ alkoxy; and
R₄ is fluoro, trifluoromethyl, nitro, C₁₋₆ alkyl, C₃₋₇ cycloalkyl, C₂₋₆ alkenyl, C₂₋₆ alkynyl, C₁₋₆ alkylthio, heteroaryl, optionally substituted aryl, optionally substituted aryl C₁₋₆ alkyl, optionally substituted aryl C₂₋₆ alkenyl, optionally substituted aryl C₂₋₆ alkynyl, optionally substituted aryloxy, optionally substituted C₁₋₆ alkoxy, optionally substituted arylthio, optionally substituted aryl C₁₋₆ alkylthio or -CONR"R''', -NR"R''', -OCONR"R''' or -SONR"R''' where R" and R"' are each hydrogen, C₁₋₆ alkyl or aryl C₁₋₆ alkyl, or R" and R''' together form a C₃₋₇ alkylene ring;
or a salt or ester thereof.

In an embodiment, R¹, R² and R³ are not all hydrogen. In another embodiment, R⁴ is not fluoro when R² and R³ are hydrogen and R¹ is hydroxy.

In the above formulae and alkyl group can be straight or branched chain, such as, for example, methyl, ethyl, propyl, isopropyl, butyl and isobutyl, and is preferably methyl or ethyl. A C₂₋₆ alkenyl group includes, for example, vinyl, prop-2-enyl, but-3-enyl, pent-4-enyl and isopropenyl. A preferred alkenyl group is of the formula R-CH=CH- where R is C₁₋₄ alkyl. An alkynyl group includes, for example, prop-2-ynyl, but-3-ynyl, and pent-t-ynyl. A preferred alkynyl group is of the formula:

R - C ≡ C -

where R is C₁₋₄ alkyl. A C₃₋₇ cycloalkyl group is preferably, for example, cyclopropyl, cyclopentyl or cyclohexyl and these groups may optionally be substiruted by one or two methyl substituents.

An aryl group is preferably phenyl or naphthyl, and an optionally substituted phenyl or naphthyl group is optionally substituted with. for example, one or more substituents, preferably 1 to 3 substituents, selected from C₁₋₄ alkyl, especially methyl, C₁₋₄alkoxy, especially methoxy and ethoxy, carboxy, hydroxy, cyano, halo, especially bromo, chloro and fluoro, trifluoromethyl, nitro, amino, C₁₋₄ acylamino and C₁₋₄ alkylthio. A napthyl group can be 1-naphthyl or 2-naphtyl. When substituted, a phenyl or naphthyl group is preferably substituted by one to three substituents. An aryl C₁₋₆ alkyl group is one such group linked through an alkylene chain, for example, aryl (CH₂)ₙ where n is 1 to 6, and a most preferred example is benzyl. Preferred examples of groups as are follows:
aryloxy - optionally substituted phenoxy;
aryl C₁₋₆ alkoxy - optionally substituted phenylmethyoxy or phenylethoxy;
arylthio - optionally substituted phenylthio;
aryl C₁₋₆ alkylthio - optionally substituted phenylmethythio or phenylethylthio.

A heteroaryl group can be aryl group having one or more hetero atoms in the ring. The term includes fused ring structures. Preferably the heteroaryl group contains one or two hetero atoms selected from oxygen, nitrogen and sulphur. It preferably contains from 5 to 10 carbon atoms, and for example may be of the formula: 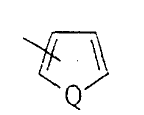 where Q is -O, -S- or -NR-, and R is hydrogen or C₁₋₄ alkyl. Alternatively, a heteroaryl group comprises a benzene fused ring as, for example: 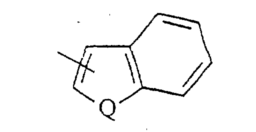 and further heteroaryl groups include:

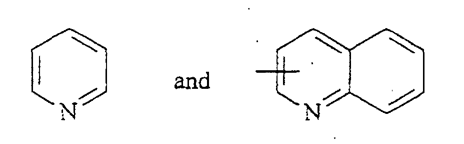

Especially preferred heteroaryl groups are pyrrolyl, thieneyl or furanyl, preferred examples being 2-thieneyl and 2-furanyl, and also pyridyl, in particular 2-and 3-pyridyl.

The group R² is preferably hydrogen, carboxy or tetrazolyl and especially carboxy, and the group R⁴ is preferably C₁₋₆ alkyl, C₂₋₆ alkenyl, optionally substituted phenyl, optionally substituted phenyl C₁₋₆ alkyl, optionally substituted phenoxy, optionally substituted phenylthio or optionally substituted phenyl C₁₋₆ alklthio.

The groups R¹ and R³ are each preferably hydrogen or hydroxy.

Examples of particular antagonists of mGluR5 that are useful in the invention include 2-methyl-6-(phenylethynyl)-pyridine (MPEP), 2-methyl-6-[(1E)-2-phenylethenyl]pyridine, 6-methyl-2-(phenylazo)-3-pyridinol, (RS)-α-methyl-4-carboxyphenylglycine (MCPG), and analogues and derivatives thereof.

An antagonist of mGluR5 may be used in a method of treatment of the human or animal body. The treatment may be a prophylactic treatment. In particular such antagonists may be used in the tolerance and/or dependence therapy. Antagonists may also be used in the manufacture of a medicament for use in tolerance and/or dependence therapy. The condition of a patient suffering from tolerance and/or dependence can be improved by administration of an antagonist of mGluR5. A therapeutically effective amount of an antagonist of mGluR5 may be given to a human patient in need thereof.

Tolerance and dependence may be treated according to the invention. Tolerance and dependence are separate phenomena.

Tolerance describes an increase in dose needed to produce a given pharmacological effect of a particular substance. In the case of opiates, for example, tolerance develops rapidly.

Dependence involves two separate components, namely physical and psychological dependence. Physical dependence is characterised by a clear-cut abstinence syndrome, such that abrupt withdrawal of a substance (cessation) may lead to, for example, increased irritability or body shakes. The exact nature of the abstinence syndrome is related to the particular substance in question. The invention may be used in the treatment of abstinence syndrome/cessation.

Psychological dependence is more complex than physical dependence and probably more important in the genesis of compulsive substance taking (ie. addiction). Typically, opiate addicts who recover fully from the abstinence syndrome are likely to revert to drug taking later. In animal models of psychological dependence on opiates based on measurement of the potentiality of drugs to act as reinforcers in tests of operant conditioning, the reinforcing effect of the drug outlasts the duration of the physical abstinence syndrome. The invention may used in the treatment of psychological dependence and in the reduction or abolition of the reinforcing effects of drugs.

The invention is applicable to the treatment of many different forms of tolerance or dependence. Tolerance or dependence may be reduced or abolished. Typically, the invention is applicable to the treatment of substance tolerance or substance dependence.

In the context of substance tolerance and dependence, the invention is applicable both to what may be loosely termed "abuse" such as nicotine addiction in the case of smokers or the consumption of other recreational drugs, and to therapeutic usage. For example, therapeutic usage of benzodiazepines and opiates may lead to tolerance to and/or dependence on those drugs. It is clearly advantageous that those consequences of pharmacological therapy effects be ameliorated or abolished.

Thus, an antagonist of a mGluR5 may be used to prevent addiction to a therapeutic pharmaceutical. In such an application, the antagonist of mGluR5 is administered before the said therapeutic pharmaceutical has itself been administered, after the said pharmaceutical has been administered or after withdrawal of the pharmaceutical, or it may be co-administered with the said pharmaceutical.

The invention is also of relevance to the treatment of a wide spectrum of other addiction-related conditions. For example, an antagonist of mGluR5 may be used in the treatment of bulimia nervosa, anorexia nervosa, betting and gambling dependence, sex dependence, sporting activity dependence or obsessive compulsive disorder. An antagonist of mGluR5 may thus be used to treat obsessive and/or compulsive behaviours and the obsessive and/or compulsive components of a variety of disorders such as gambling and/or compulsive sexual activity.

The invention provides treatment of tolerance to and dependence on a number of substances. The invention is particularly useful in the treatment of dependence on nicotine, for example to treat withdrawal effects of smoking cessation. Thus, the invention may be used in the treatment of smoking cessation-related phenomena including craving, depression, anxiety, concentration difficulty and weight gain. Withdrawal symptoms associated with smoking cessation can be reduced.

The invention is also useful in the treatment of cocaine, amphetamine and alcohol addiction. Dependence on cocaine, amphetamines and alcohol, may be reduced or abolished. Tolerance of and dependence on amphetamine-related drugs such as dextroamphetamine, methylamphetamine, methylphenidate and lenfluramine may also be treated using an antagonist of mGluR5.

The invention is further useful in the treatment of opiate tolerance or dependence, in both "abuse" contexts, for example dependence on heroin and pharmaceutical contexts, for example in the prophylaxis of morphine tolerance and/or dependence. Additionally. tolerance and dependence on benzodiazepines, including diazepam and temazepam may be treated by the use of an antagonist of mGluR5.

Antagonists of mGluR5 may be administered in a variety of dosage forms. Thus, they can be administered orally, for example as tablets, troches, lozenges, aqueous or oily suspensions, dispersible powders or granules. The antagonists may also be administered parenterally, either subcutaneously, intravenously, intramuscularly, intrasternally, transdermally or by infusion techniques. The inhibitors may also be administered as suppositories. A physician will be able to determine the required route of administration for each particular patient.

The formulation of an antagonist of mGluR5 will depend upon factors such as the nature of the exact antagonist, whether a pharmaceutical or veterinary use is intended, etc. An antagonist of mGluR5 may be formulated for simultaneous, separate or sequential use.

An antagonist of mGluR5 is typically formulated for administration in the present invention with a pharmaceutically acceptable carrier or diluent. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents: starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain. together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

A therapeutically effective amount of an antagonist of mGluR5 is administered to a patient. The dose of an antagonist of mGluR5 may be determined according to various parameters, especially according to the substance used; the age, weight and condition of the patient to be treated; the route of administration; and the required regimen. Again, a physician will be able to determine the required route of administration and dosage for any particular patient. A typical daily dose is from about 0.1 to 50 mg per kg of body weight, according to the activity of the specific inhibitor, the age, weight and conditions of the subject to be treated, the type and severity of the degeneration and the frequency and route of administration. Preferably, daily dosage levels are from 5 mg to 2 g.

The invention also provides methods for identifying products which may be used in a method of treatment of the human or animal body by therapy, in particular in the treatment of tolerance or dependence. Such methods essentially comprise determining whether a test product is a mGluR5 antagonist and determining whether an antagonist so-identified can be used in the treatment of substance tolerance or dependence.

Antagonists of mGluR5 are defined above and any suitable assay may be used may be carried out in order to determine whether a test product is an mGluR5 antagonist. Preferably, any assay used will be suitable for high through-put screening, for example the assay will ideally be carried out in a single well of a plastics microtitre plate.

Any suitable assay format may be used for identifying an antagonist of mGluR5. Preferably assay formats are adapted so that they can be carried out in a single well of a plastics microtitre plate. Thus, assays may be adapted for use in high through-put screening techniques.

One example of an assay for determining the activity of a test compound as an antagonist of mGluR5 comprises expressing mGluR5 in CHO cells which have been transformed with cDNAs encoding the mGluR5 receptor protein (Daggett *et al*., 1995, Neuropharmacology **34**, 871). The mGluR5 is then activated by the addition of quisqualate and/or glutamate and can be assessed by, for example the measurement of : (i) phosphoinositol hydrolysis (Litschig *et al*., 1999, Mol. Pharmacol. **55**, 453); (ii) accumulation of [3H] cytidinephosphate-diacylglycerol (Cavanni *et al*., 1999, Neuropharmacology **38**, A10); or fluorescent detection of calcium influx into cells (Kawabata *et al*., 1996, Nature **383**, 89; Nakahara *et al*., 1997, J. Neurochemistry **69**, 1467). The assay is be carried out both in the presence and absence of a test product in order to determine whether the test compound can antagonise the activity of the test product.

Suitable control experiments can be carried out. For example, a putative antagonist of mGluR5 could be tested with mGluR1 in order to determine the specificity of the putative antagonist, or other receptors unrelated to mGluRs to discount the possibility that it is a general antagonist of cell membrane receptors.

Suitable test products for identifying a mGluR5 antagonist include combinatorial libraries, defined chemical identities, peptides and peptide mimetics, oligonucleotides and natural product libraries. The test products may be used in an initial screen of, for example, ten products per reaction. and the products of batches which show antagonism tested individually. Furthermore, antibody products (for example, monoclonal and polyclonal antibodies, single chain antibodies, chimeric antibodies and CDR-grafted antibodies) may be used.

Test products which show activity in assays such as those described above can be tested in *in vivo* systems, such as an animal models of substance, for example nicotine, cocaine or amphetamine dependence. Appropriate models are described in the Examples below.

The invention thus provides products identified by a method for identifying a product which may be used in a method of treatment of the human or animal body by therapy, in particular in the treatment of tolerance or dependence. Such products may be used in methods of treatment as described above.

The following Examples illustrate the invention:

### Example 1

### Introduction

It has been widely reported that smoking-related cues play a major role in the maintainence of tobacco smoking, craving for cigarette, difficulty to remain abstinent and in relapse to smoking. Human laboratory investigations have shown that smoking related cues acquire their conditioned value from the association with nicotine reinforcement obtained from cigarette smoking. Nicotine self-administration in laboratory animals is a well-established models of nicotine reinforcing properties, where specific nicotine-taking and nicotine-seeking behaviours can be induced and maintained for several weeks. Dopaminergic antagonism or lesions of dopamine pathways have been shown to reduce nicotine self-administration.

This model was shown to possess face validity when mimicking the behavioural effects due to determinant factors of smoking relapse: the re-exposure to nicotine or to a stressful condition is able to reinstate nicotine-seeking behaviour in rats after long-term nicotine abstinence and extinction of nicotine self-administration behaviour: Chiamulera *et al*., Psychopharmacology, 127, 102 (1996); Buczek *et al*., Psychopharmacology, 144, 183 (1999). A protocol where it is possible to induce relapse in rats by exposing them to determinants of relapse after a short-term abstinence, is describe in Chiamulera et al., Arch. Pharmacol., 358, R578 (1998).

### Results

The compound 2-methyl-6-(phenylethynyl)-pyridine (MPEP) is a selective and potent antagonist of mGluR5 receptors as assessed on recombinant mGluR5 receptors compared with ionotropic glutamate receptors and other mGluRs. The pharmacology of this compound has been characterised in cell lines, cell cultures, in vitro pharmacological assays, and. systemically-given in vivo electrophysiology.

MPEP was given to rats in order to assess the effect against nicotine-seeking relapse in a model already validated with current (nicotine preload) or potential (mecamylamine) smoking cessation pharmacotherapy. Male Wistar rats (Charles River) maintained 240g-260g (85% of their ad libitum body weight) were used. Animals were individually housed in a temperature-controlled room on a 12-h light/12-h dark cycle.

Rats were trained to lever press for nicotine infusion (0.03 mg/kg/infusion) in operant Skinner boxes. Nicotine was dissolved in heparinized saline (0.09% NaCl + 0.5 UI/ml heparine) and pH adjusted to 7.4 with NaOH. Nicotine doses are expressed as mg of free base/kg of body weight per infusion. After acquisition of the operant behaviour, rats were maintained on a daily schedule of nicotine self-administration for at least 2-3 weeks. In these experimental conditions, a stable performance of drug-taking behaviour (measured as number of nicotine infusions or number of nicotine-paired lever presses for session) was the criteria for the acquisition and maintenance of nicotine dependence.

On test day, 24 hours after the last nicotine self-administration session, rats were exposed to a multiple "relapse schedule" consisting of two components: firstly, a 30-min phase with exposure to the context (chamber) and, contingentlyr upon responding, to conditioned stimuli (tone + cue lamp) previously paired to nicotine self-administration. At the end of the first component, a non-contingent subcutaneous (s.c.) injection of nicotine 0.15 mg/kg was delivered to rats as priming and nicotine-paired lever presses (but without consequences upon responding) were measured during the second 120-min phase ("nicotine component"). The s.c. nicotine injection induced a reinstatement of responding significantly greater than after saline injection.

Pharmacological characterization of this protocol has been performed using two standard treatments for smoking cessation (Rose & Corrigall, Psychopharmacology, 130, 28, 1997). Pretreatment with mecamylamine (1mg/kg s.c. given immediately prior starting the relapse session) or nicotine preload (0.03 mg/kg i.v., given 30 min prior session) but not corresponding vehicles, were able to significantly reduce nicotine-paired lever presses during the "nicotine component" (respectively -68% and -49% compared to responding after vehicle treatment; P=<0.05 Student's t test). However, no significant effect on responding during the "cue component" was observed.

MPEP was dissolved in saline (0.09% NaCl) and given to rats at the doses of 1, 10 mg/kg or vehicle intravenously, single bolus, in a volume of 1 ml/kg body weight, 5 minutes prior the start of the relapse test session. Testing drug pretreatment effects was done on different test session days. At least two stable self-administration sessions elapsed between the different relapse test sessions.

MPEP induced inhibition of reinstatement of responding for nicotine-paired lever at both doses 1 and 10 mg/kg (respectively -60% and -86% compared to responding after vehicle treatment; P=<0.05 ANOVA) during the "cue component". Both doses were also significantly effective to reduce reinstatement during the "nicotine component" phase at all the time-points, with a maximal effect measured at the 90^{th} minute: respectively -42% and -98% compared to responding after vehicle treatment; P=<0.05 ANOVA.

Thus we have demonstrated that a compound which acts as a selective antagonist of mGluR5 is capable to reduce the reinstatement of nicotine-seeking behaviour in rats following exposure to experimental determinants of relapse. It is thought that the pharmacological blockade of mGluR5 receptors can negatively modulate D2 receptor and therefore reduce the dopaminergic activity which underlying drug-seeking behaviours, including smoking relapse.

### Example 2

### Introduction

In order to investigate the mechanisms underlying cocaine dependence, we have studied the physiological and behavioural effects of cocaine on mGluR5 knockout mice. In order to take in consideration possible mouse strain variations, the knockout has been separately derived in two inbred strains: C57Black/6 and 129Sv. Studies presented here were performed in this two strains.

### Materials and Methods

*Gene targeting*: mGluR5 knockout mice were generated as follows: a genomic fragment of 8.5 kb containing the last exon of mouse mGluR5 gene was isolated from an ES cell-isogenic DNA library. A neomycin resistance cassette and'a TK selection unit were inserted according to standard procedures into the two Sac II sites of the last exon, resulting in a deletion of 360 bp in the coding sequence of the mGluR5 gene. Mutation was detected by Southern blot by cutting the genomic DNA with EcoR I resulting in a WT band of 5.1 kb and a mutated band of 2.2 kb after hybridization with an internal probe. Heterozygous and homozygous animals were then generated as previously described²³ and the absence of mGluR5 was confirmed with a mGluR5 antibody (Upstate Biotech). In addition, heterozygotes were separately crossed with inbred C57B1/6J and 129SvPasIco mice for 5 generations. Mutant homozygotes as well as WT mice were then obtained by crossing heterozygotes of the fifth generation within each background. Studies presented here were performed with adult WT (+/+) and KO (-/-) mice from these crossings.

*Cocaine self-administration:* Male mGluR5 (+/+) and (-/-) mice were housed in a temperature controlled vivarium on a 12/12 hour dark/light cycle (lights on at 7:00am) with food and water available ad libitum except during behavioral training and testing. All food training and drug self-administration procedures were completed in operant chambers (MedAssociates, Georgia, VT., USA) equipped with 2 levers (one active, the other inactive; counterbalanced between subjects) on one wall and with a liquid dipper located in the center of the opposite wall. Experimental parameters and data collection were controlled by an IBM compatible PC using MedPC for Windows software (Med Associates, Georgia. VT., USA). Intravenous injections were delivered by an infusion pump (Model A, Razel Scientific) through Tygon tubing (0.02 inch inner diameter (i.d.) x 0.06 inch outer diameter (o.d.), 0.02 inch wall) connected on one end to a stainless steel swivel and counterbalance assembly (Instech, King of Prussia, PA., USA) to allow free movement of the animal, and on the other end to the catheter base mounted in the midscapular region of the mouse.

Mice were initially trained to press a lever to earn a liquid reinforcer (whole milk with sucrose, 60 g/L) under a fixed ratio 1 time-out 1 second schedule of reinforcement. After mice earned 30 reinforcers during a 1-hour session under this schedule, the schedule requirements were increased across sessions up to a fixed-ratio 2 time-out 20 second schedule, which is identical to the reinforcement schedule used subsequently during cocaine self-administration. This training typically decreases the time required for animals to acquire intravenous self-administration and also allows for the assessment of differences in learning rate and/or ability to perform the operant task separate from the assessment of the reinforcing effects of drugs.

After acquisition of the lever press task, mice were anesthetized with an oxygen-halothane mixture (0.8-1.5% halothane) and implanted with chronic intravenous jugular catheters as described previously (Caine *et al*. 1999, Psychopharmacology **147**,22-24; Deroche *et al*. 1997, Pharmacol. Biochem. Behav. **57**, 429-40). Catheters consisted of Silastic tubing attached to a stainless steel guide cannula (Plastics One, Roanoke. VA) bent at a right angle and encased in dental cement anchored with a 1.0 cm square of soft cloth mesh. The tubing was passed subcutaneously from the animals mid-scapular region to the right external jugular vein, where it was inserted and secured with suture thread. Animals were allowed at least 48 hours recovery from surgery before being given access to cocaine. To ensure patency, catheters were flushed daily with approximately 0.01 - 0.03 ml saline containing heparin (30 usp units/ml). When not in use, catheters were capped with a short length of Tygon tubing plugged with monofilament. Catheter patency was tested with Brevital Sodium (1% methohexital sodium, Eli Lilly, Indianapolis, IN) at the end of the self-administration experiment to verify catheter patency. Animals with patent catheters exhibit pronounced loss of muscle tone within 2 seconds of intravenous (iv) injection of Brevital.

All mice were trained with cocaine (0.8 mg/kg/injection, 50 µl over 2 seconds) where 2 lever presses on the active lever activated the infusion pump and delivered a single injection of drug solution under a fixed ratio (FR) 2 schedule with a 20 second time-out (TO20s) period (during which lever presses were recorded but had no programmed consequences) until stable baseline was reached (3 consecutive sessions with < 20% variation from the mean and > 75% active nose-poke responding). After mice achieved stable baseline self-administration, a cocaine dose response curve was determined in which mice were given access to various doses of cocaine (0.0, 0.4, 0.8, 1.6, and 3.2 mg/kg/inj.) during single daily sessions in a Latin square design. The number of cocaine injections earned per session at each dose was recorded and the number of injections at each dose was determined during at least 2 separate sessions at each dose.

Because mGluR5 (-/-) mice failed to acquire stable cocaine self-administration and instead their lever pressing extinguished during 3-5 sessions of access to cocaine, they were retrained to lever press for food to criterion (30 food reinforcers per session) in between each cocaine dose. This procedure was instituted so that the mGluR5 (-/-) mice had a non-zero response rate during the first couple sessions of access to each dose of cocaine. Typically this food retraining required only 1 or 2 sessions before testing resumed with the subsequent cocaine dose. mGluR5 (-/-) mice were tested at the same doses of cocaine as the mGluR5 (-/+) mice, in a Latin square dose order and were tested at each dose until responding stabilised below 5 injections per session.

*Microdialysis*: The transcerebral microdialysis technique was used to measure DA levels in theNucleus Accumbens of the wild as well as of the ko mice. Animals were anesthetized with chloral hydrate (450 mg/kg/10 ml ip.) and placed in a stereotaxic apparatus for small animals by means of the snout adaptor only. The skull was exposed and the lateral muscles were cut and displaced in order to show the parietal bones. A 1-mm-hole was drilled on each side and a previously prepared orizontal microdialysis probe (Zocchi *et al*. 1998. Neuroscience **82**, 2) was inserted in one hole by a micromanipulator and pushed all the way across the brain to exit at the opposite hole. Coordinates were calculated to position the probe at the level of the Nucleus Accumbens (A: +2.2 mm, V: -4.6 from bregma). The ends of the probe were glued to 5-mm-long stainless steel cannulae and secured on top of the skull with epoxy glue. Skin was sutured to leave a slot to allow the passage of the cannulae. The animal was singly housed for at least 24 hr before starting the experiment.

On the day of the experiment the cannulae were connected to poliethylen tubing (PE 10) by which an artificial cerebrospinal fluid (KCl 4mM, NaCl 147 mM, CaCl₂ 1.3 mM) was pumped through the probe at a steady flow rate of 1 µl/min. Tubing was connected to a liquid swivel to allow the animal free movement. Four hours of perfusion were allowed to pass before injecting ip. the treatment. Samples were collected every 20 min for 3 hours following the treatment and immediately frozen at -80 °C. Localization of the probe was checked in each animal at the end of the experiment using a criostat and a magnifying lens.

### Results

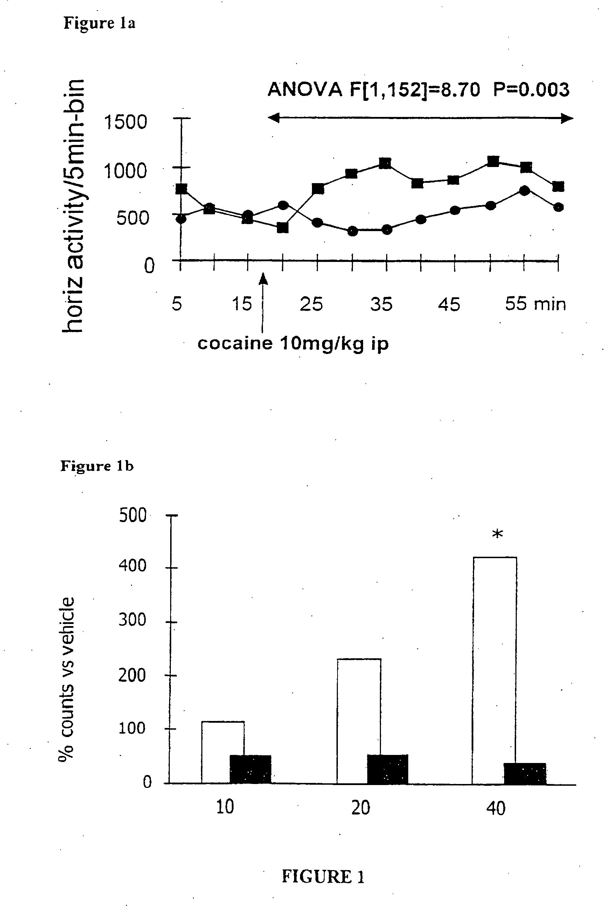

Basal level of locomotor activity of mGluR5(-/-) mice was not significantly different from the wild type mice as revealed by habituation tests (not shown). In a preliminary experiment. when mice were treated with the dopamine re-uptake inhibitor cocaine (10mg/kg, ip) the results obtained showed that mutant mice did not have the expected increase of locomotion (Fig. 1a). A definitive experiment was done by using different doses of cocaine in order to characterised the reduced mutant sensitivity to the stimulants effects of cocaine. Mice from both groups were treated with cocaine 10, 20, 40 mg/kg i.p. or vehicle on different test days along a randomised order of dosing (i.e. all the subjects received all treatments). Cocaine induced a dose-related increase in horizontal activity in the wild-type but not in the mutant group of mice (Fig. 1b) confirming the previous findings. Furthermore, an evoked 4 mg/kg intravenous injection of cocaine did not induce any apparent effect on the knockout animals. Therefore, our findings suggest that mGluR5 is essential for cocaine-induced hyperactivity.

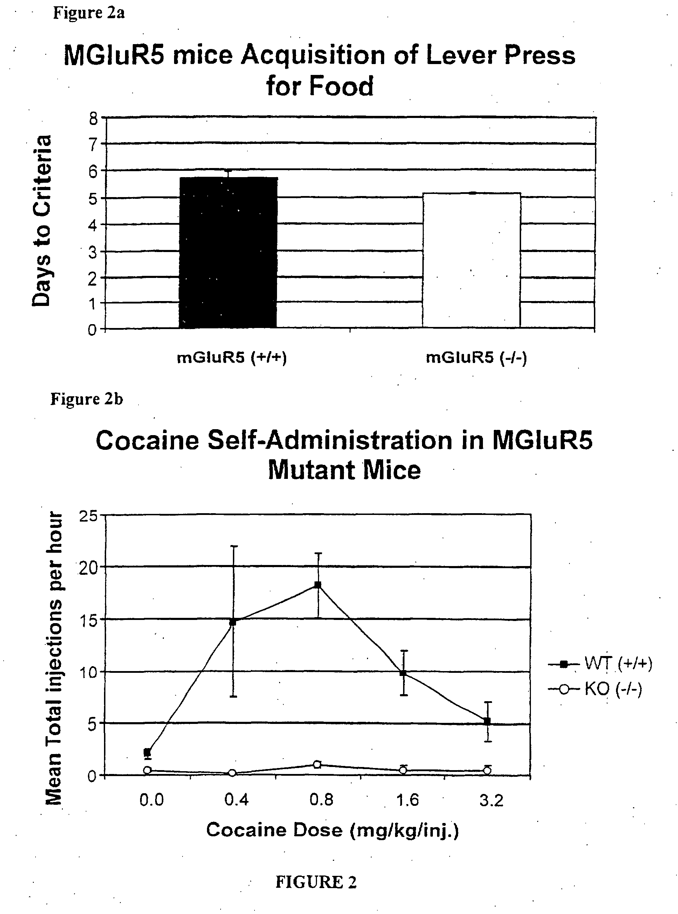

It is well known that psychostimulant-induced hyperactivity does not reflect reinforcing effects of cocaine which is the main cause of drug addiction (Koob *et al*., 1998, Neuron, 21. 467-476). In order to better assess this latter effect, we have applied the intravenous (IV) cocaine self-administration method to both mGluR5 mutant and wild-type mice (Epping-Jordan *et al*., 1998, Brain Research, **784**, 105). Animals were first trained with food in order to allow them to acquire an operant behaviour which consists in pressing an active lever in defined conditions. As shown in Fig.2a, both mutant and wild type animals acquired successfully and at the same rate the task of food intake prior to cocaine self-administration. This was an . important control experiment since some learning impairment has been reported in mGluR5 (-/-) mice (Lu *et al*., 1997, J. Neurosci. **17**, 5196-5205.). If wild-type mice self administered cocaine at 0.8 mg/kg in a stable manner (i.e. after 6 days), mGluR5 (-/-) mice showed an extinction of the response and stopped pressing the active lever after three sessions. Moreover, mGluR5 knockout mice did not self-administer cocaine at any tested dose (from 0.4 to 3.2 mg/kg/injection) while wild-type mice showed a standard dose-response curve with an optimal dose response curve with a maximum at 0.8 mg/kg (Fig.2b). The results from the food training suggest that the failure to acquire cocaine self-administration in the mGluR5 (-/-) mice is not due to an inability to learn the operant lever-press task. The results of the cocaine self- i administration experiments suggest that the reinforcing properties of cocaine are absent in mice lacking the gene for mGluR5. Then, in addition to hyperactivity response, mGluR5 may also be an essential component of the reward process induced by cocaine.

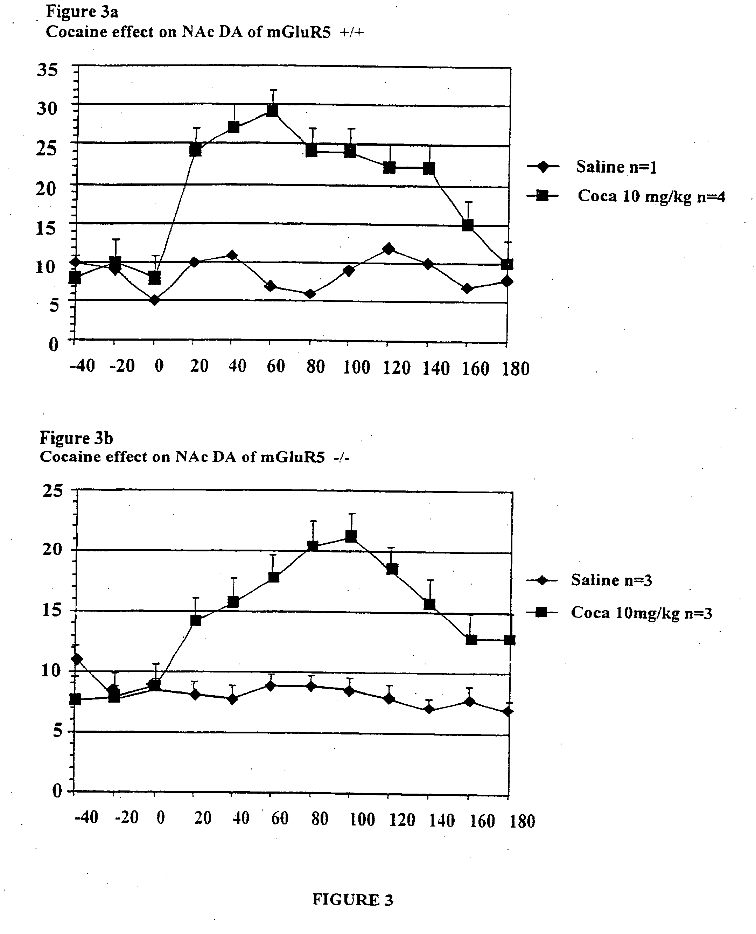

Several studies have reported that treatment with psychostimulants induced a release of DA in the ventral striatum of rodents (Koob *et al*. 1998, *supra*). In addition, a close correlation has been established between locomotor activity and mesoaccumbens DA release in response to psychostimulants (Zocchi *et al*. 1998, *supra*). Because mGluR5(-/-) mice did not show neither a locomotor response nor an addictive behaviour for cocaine, we examined the level of DA release in the nucleus accumbens of wild type and mutant mice after cocaine treatment (MD). As shown in Fig. 3, microdyalisis experiments revealed that levels of DA release were similar in the nucleus accumbens of both control and knockout mice after 10 mg/kg cocaine IP-injection, suggesting that the absence of mGluR5 does not affect neither the presynaptic release of mesoaccumbens DA, nor the function of the DA transporter (DAT) since both curves show the same peak and the same decrease of DA concentrations (Fig. 3). Furthermore, historadiography studies performed on the two genotypes revealed no differences in DA binding levels suggesting no change in the expression of DA receptors in the mutant mice (not shown).

Recent studies have shown that activation of mGluRs in the nucleus accumbens increased not only DA release but also induced DA-dependent locomotor activation (Attarian and Amalric, 1997, Eur. J. Neurosci., **9**, 809; Kim and Vezina, 1997, J. Phar. Exp. Ther., **283**, 962; Vezina and Kim, Neurosci. Behav. Rev., **23**, 577). This effect has been shown to be blocked by the non-selective group I mGluR antagonist α-methyl-4-carboxyphenylglycine (MCPG). These results suggest an interaction between mGlu receptors and Dopaminergic transmission. Since, as reported here, the absence of mGluR5 does not affect DA release in the nucleus accumbens of mutant mice, mGluR5 may then exert a control from the postsynaptic side of the synapse on the Dopaminergic activity. Hence, this receptor has been shown to be located - as group I mGluRs - postsynaptically (Romano et *al*. 1995, Comp. Neurol., **355**, 3). On the other hand, the interaction with DA release described above, might be mediate by group II or III mGluRs, which are known to control synaptic activity from the presynaptic side of the synapse.

Research has been mainly focused on the understanding of the contribution of Dopaminergic and Serotoninergic systems in drug dependence (Koob *et al*. 1998, *supra).* However, recent studies on DA receptors and DA transporter (DAT) knockout mice gave unexpected results (see Draso *et al*. 1998, Dev. Neurosci, **20**, 2-3 for review). D2 receptor mutant mice have an abnormal locomotor spontaneous activity but still show a locomotor response to IP-injections of opioids (Maldonado *et al*. 1997, Nature, **388**). In addition, it is noteworthy that D1 receptor or DAT knockout mice for which no increase in locomotor activity was observed after cocaine injection, had also an abnormal spontaneous behaviour (Miner *et al*. 1995, Neuroreport, **6** ; Giros *et al*., 1996, Nature, **379**). In contrast, mGluR5 mutant mice are not spontaneously hyperactive and still, showed an absence of locomotor response to cocaine in both backgrounds C57B16 and 129Sv (Fig. 1). Therefore, it can be unambiguously stated that cocaine does not increase locomotor activity in the mGluR5 knockout mice.

The fact that the mutant mouse for the DAT gene, while hyperactive, still self-administers cocaine, suggests that the Dopaminergic transmission may not be the main effector of reward process induced by cocaine reinforcement (Rocha *et al*. 1998, Nature Neurosci. **1**, 2). In contrast, results presented here showing that mGluR5 knockout mice do not self-administer cocaine at any tested dose strongly suggest that mGluR5 plays a critical role in reward mechanisms. Because the mutation is carried over the embryogenesis of the mouse, some changes may have occured in neuronal development which in turn might have important consequences in the onset of the reward process in adulthood. Nevertheless. the fact that DA release is not affected in the nucleus accumbens of mGluR5 (-/-) mice as shown by microdyalis studies, suggests that the dopaminergic response at least at this level, is preserved in the mutant. It is noteworthy that this result is in contradiction with a well admitted statement according to which elevated DA is a mandatory condition for cocaine reinforcing effects (Koob *et al*. 1998, supra). It is possible that mGluR5 act in synergy with dopaminergic system and at the postsynaptic side of the neuron to mediate reward mechanisms as well as hyperactivity response induced by cocaine. Hence, a cooperative activity between Dopamine receptors and metabotropic glutamate receptors has been recently described in the prefrontal cortex of mice (Otani *et al*., 1999, J. Neurosci. **19**, 9788-9802). Put together with results from other knockout studies, our data emphasizes the importance of glutamate as one of the main neurotransmitter of drug of abuse and mGluR5 as a crucial element of reward process.

In summary, we have shown that, while having no apparent phenotype, mGluR5 knockout mice showed an absence of locomotor response to cocaine injection. In addition, mGluR5(-/-) mice did not self administer cocaine at any tested dose whereas mGluR5(+/+) mice showed a standard dose response curve. Finally, after cocaine treatment, Dopamine (DA) was similarly released in the nucleus accumbens of both mutant and wild type mice suggesting a signal transduction-mediated effect of mGluR5 on Dopaminergic system. Our results demonstrate that, in addition to dopamine, glutamate through mGluR5 plays a crucial role in dependence to psychostimulants.

### Example 3: d-amphetamine self-administration

All housing, training, surgical and testing conditions for experiments of d-amphetamine self-administration were identical to those used in experiments of cocaine self-administration, described in Example 2 above. Male mGluR5 (-/-) mice were initially tested for d-amphetamine self-administration (0.2 mg/kg/injection) under a fixed ratio (FR) 2 schedule with a 20 second time-out (TO20s).

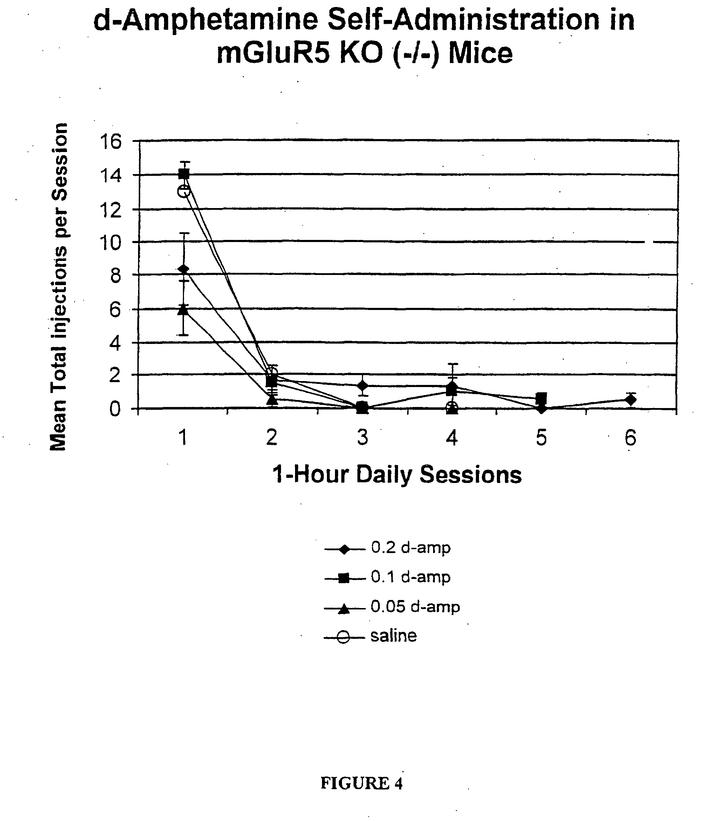

The results are shown in Figure 4. All mice tested failed to acquire self-administration at this initial dose. Mice were also tested at doses of 0.1, 0.05 and 0.0 (saline) mg/kg/injection d-amphetamine and showed near complete extinction of responding at all doses, including saline within five sessions. As in the cocaine self-administration experiments, because mGluR5 (-/-) mice failed to acquire stable d-amphetamine self-administration, they were retrained to lever press for liquid reinforcers to criterion in between each d-amphetamine dose. This procedure was instituted so that mGluR5 (-/-) mice had a non-zero response rate during the first j couple sessions of access to each dose of d-amphetamine. The failure of mGluR5 (-/-) mice to acquire d-amphetamine self-administration suggests that mGluR5 has an essential role in the reinforcing properties of d-amphetamine.

## Claims

1. Use of an antagonist of the metabotropic glutamate receptor 5 (mGluR5) in the manufacture of a medicament for use in a method of tolerance or dependence therapy.

2. Use according to claim 1 in the treatment of smoking dependence

3. Use according to claim I in the treatment of substance tolerance or dependence.

4. Use according to claim 1 in the treatment of substance withdrawal or cessation.

5. Use according to claim 3 or 4, wherein the substance is nicotine, cocaine, amphetamine or related drugs thereto, a benzodiazepene, an opiate or ethanol.

6. Use according to claim 3 or 4, wherein the substance is a therapeutic substance.

7. Use according to claim 6, wherein the therapeutic substance is amphetamine or related to drugs thereto, a benzodiazepene or an opiate.

8. Products containing an antagonist of mGluR5 and a therapeutic substance as a combined preparation for simultaneous, separate or sequential use in the treatment of a condition for which the said therapeutic substance is used, wherein the use of the therapeutic substance in the absence of said antagonist could lead to tolerance of or dependence on the therapeutic substance.

9. Use of a product identified by a screening method comprising:
(a) contacting a test product with mGluR5 under conditions that in the absence of the test substance would lead to activity of the said mGluR5; and
(b) determining whether the test product antagonises mGluR5 activity, thereby to determine whether the test product may be used in the treatment of substance tolerance or dependence,
for the manufacture of a medicament for use in tolerance or dependence therapy.

10. products containing a product identified by a screening method as defined in claim 9 and a therapeutic substance as a combined preparation for simultaneous, separate or sequential use in the treatment of a condition for which the said therapeutic substance is used, wherein the use of the therapeutic substance in the absence of said product could lead to tolerance of or dependence on the therapeutic substance.

11. Use of an antagonist of the metabotropic glutamate receptor 5 (mGluR5) in the manufacture of a medicament for use in the treatment of bulimia nervosa, anorexia nervosa, gambling dependence, sex dependence or obsessive compulsive disorders.

## Patentansprüche

1. Verwendung eines Antagonisten des metabotropen Glutamatrezeptors 5 (mGluR5) in der Herstellung eines Medikaments zur Verwendung in einem Verfahren der Gewöhnungs- oder Abhängigkeitstherapie.

2. Verwendung gemäss Anspruch 1 in der Behandlung der Abhängigkeit vom Rauchen.

3. Verwendung gemäss Anspruch 1 in der Behandlung von Gewöhnung an oder Abhängigkeit von Substanzen.

4. Verwendung gemäss Anspruch 1 in der Behandlung des Entzugs oder des Absetzens von Substanzen.

5. Verwendung gemäss Anspruch 3 oder 4, worin die Substanzen Nikotin, Kokain, Amphetamin oder damit verwandte Wirkstoffe, ein Benzodiazepin, ein Opiat oder Ethanol ist.

6. Verwendung gemäss Anspruch 3 oder 4, worin die Substanzen ein Therapeutikum ist.

7. Verwendung gemäss Anspruch 6, worin das Therapeutikum Amphetamin oder damit verwandte Wirkstoffe, ein Benzodiazepin oder ein Opiat ist.

8. Erzeugnisse, die einen Antagonisten von mGluR5 und ein Therapeutikum als Kombinationszubereitung zur gleichzeitigen, separaten oder aufeinanderfolgenden Verwendung in der Behandlung eines Zustands enthalten, für den das Therapeutikum verwendet wird, worin die Verwendung des Therapeutikums in Abwesenheit des Antagonisten zu einer Gewöhnung an oder einer Abhängigkeit von dem Therapeutikum führen könnte.

9. Verwendung eines Erzeugnisses, das durch ein Durchmusterungsverfahren identifiziert wird, welches umfasst:
(a) In-Kontakt-Bringen eines Testerzeugnisses mit mGluR5 unter Bedingungen, die in Abwesenheit des Testerzeugnisses zu Aktivität des mGluR5 führen würden; und
(b) Bestimmen, ob das Testerzeugnis der mGluR5-Aktivität entgegenwirkt, um dadurch zu bestimmen, ob das Testerzeugnis in der Behandlung von Gewöhnung an oder Abhängigkeit von Substanzen verwendet werden kann,
zur Herstellung eines Medikaments zur Verwendung in der Gewöhnungs- oder Abhängigkeitstherapie.

10. Erzeugnisse, die ein Erzeugnis, das durch ein Durchmusterungsverfahren wie in Anspruch 9 definiert identifiziert wurde, und ein Therapeutikum enthalten, als Kombinationszubereitung zur gleichzeitigen, separaten oder aufeinanderfolgenden Verwendung in der Behandlung eines Zustands, für den das Therapeutikum verwendet wird, worin die Verwendung des Therapeutikums in Abwesenheit des Erzeugnisses zu einer Gewöhnung an oder Abhängigkeit von dem Therapeutikum führen könnte.

11. Verwendung eines Antagonisten des metabotropen Glutamatrezeptors 5 (mGluR5) in der Herstellung eines Medikaments zur Verwendung in der Behandlung von Bulimia nervosa, Anorexia nervosa, Spielsucht, Geschlechtssucht oder Zwangsstörungen.

## Revendications

1. Utilisation d'un antagoniste du récepteur 5 du glutamate métabotropique (mGluR5) pour la fabrication d'un médicament utilisé dans la thérapie de l'accoutumance ou de la dépendance.

2. Utilisation selon la revendication 1, dans le traitement de la dépendance vis-à-vis du tabagisme.

3. Utilisation selon la revendication 1, dans le traitement de l'accoutumance ou de la dépendance vis-à-vis d'une substance.

4. Utilisation selon la revendication 1, dans le traitement du sevrage ou de l'arrêt de la prise d'une substance.

5. Utilisation selon la revendication 3 ou 4, dans laquelle la substance est la nicotine, la cocaïne, une amphétamine ou des drogues apparentées, une benzodiazépine, un opiacé ou l'éthanol.

6. Utilisation selon la revendication 3 ou 4, dans laquelle la substance est une substance thérapeutique.

7. Utilisation selon la revendication 6, dans laquelle la substance thérapeutique est une amphétamine ou des drogues apparentées, une benzodiazépine ou un opiacé.

8. Produits contenant un antagoniste de mGluR5 et une substance thérapeutique sous la forme d'une préparation combinée pour l'utilisation simultanée, séparée ou successive dans le traitement d'un état pour lequel ladite substance thérapeutique est utilisée, où l'utilisation de la substance thérapeutique en l'absence dudit antagoniste pourrait conduire à une accoutumance ou à une dépendance vis-à-vis de la substance thérapeutique.

9. Utilisation d'un produit identifié par une méthode de criblage, comprenant :
(a) la mise en contact d'un produit de test avec mGluR5 dans des conditions qui, en l'absence de la substance de test, conduiraient à l'activité dudit mGluR5 ; et
(b) la détermination du fait de savoir si le produit de test contrarie l'activité de mGluR5, de façon à déterminer ainsi si le produit de test peut être utilisé dans le traitement d'une accoutumance ou d'une dépendance vis-à-vis d'une substance,
pour la fabrication d'un médicament utilisé dans une thérapie de l'accoutumance ou de la dépendance.

10. Produits contenant un produit identifié par une méthode de criblage selon la revendication 9, et une substance thérapeutique sous la forme d'une préparation combinée pour l'utilisation simultanée, séparée ou successive dans le traitement d'un état pour lequel ladite substance thérapeutique est utilisée, où l'utilisation de la substance thérapeutique en l'absence dudit produit pourrait conduire à une accoutumance ou à une dépendance vis-à-vis de la substance thérapeutique.

11. Utilisation d'un antagoniste du récepteur 5 de glutamate métabotropique (mGluR5) pour la fabrication d'un médicament utilisé dans le traitement de la boulimie nerveuse, de l'anorexie nerveuse, de l'addiction au jeu, de l'addiction sexuelle ou de troubles obsessionnels compulsifs.
